Europäisches Patentamt

European Patent Office

Office européen des brevets

(11) Publication number: **0 094 317**
**A2**

# EUROPEAN PATENT APPLICATION

(21) Application number: **83400938.3**

(51) Int. Cl.³: **C 12 P 21/00**

(22) Date of filing: **09.05.83**

(30) Priority: **12.05.82 JP 80697/82**

(43) Date of publication of application: **16.11.83**
**Bulletin 83/46**

(84) Designated Contracting States: **CH DE FR IT LI NL SE**

(71) Applicant: **SHIONOGI & CO., LTD., 12,
Dosho-machi 3-chome Higashi-ku, Osaka 541 (JP)**

(72) Inventor: **Kumagai, Katsuo, 6-25-3, Sakuragaoka,
Sendai-shi Miyagi (JP)**

(74) Representative: **Fruchard, Guy et al,
NOVAPAT-CABINET CHEREAU 107, boulevard Péreire,
F-75017 Paris (FR)**

(54) **Production of interleukin and its analogous immune regulating factors.**

(57) Interleukin-2 and macrophage activating factor (MAF) are produced by incubation of the human peripheral blood cells in the presence of the human lymphoblast cells and an interleukin inducer. Interleukin-2 and MAF are useful in treatment of infectious diseases caused by virus or bacteria or of malignant tumors. The yield is greatly increased by the present method.

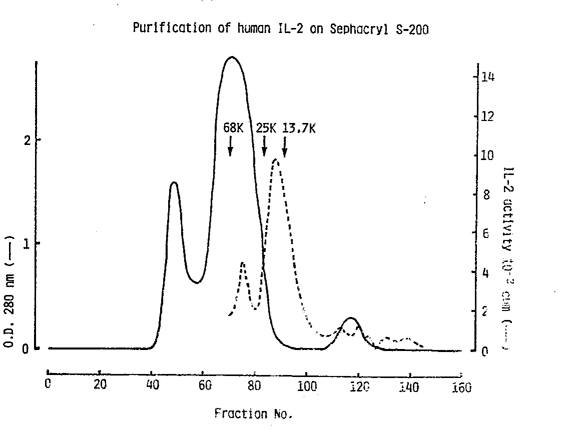

Purification of human IL-2 on Sephacryl S-200

## Background of the Invention

The present invention relates to a process for producing interleukin and its analogous immune regulating factors by utilizing human peripheral blood cells.

### 1. Field of the Invention

The term interleukin is a generic name of a group of substances which are produced by macrophage and lymphocyte, presently known as interleukin-1 produced by the macrophage, and interleukin-2 and interleukin-3 produced by the lymphocyte. The macrophage and lymphocyte in addition to the above substances produce many liquid factors relating to immune; particularly, the macrophage activating factor which accelerates differentiation of macrophage and has been called lymphokine generally, is included in the interleukin in a broad sense. When the living body is infected by virus or bacteria or influenced by growth of cancer, the macrophage and lymphocyte produce the interleukin (or lymphokine) in the body fluid on stimulation with those antigens. They are essential components in the living body required for immune response.

### 2. Description of the Prior Art

In order to produce interleukin from the human lymphocyte or macrophage by artificial induction, there is a method in which the human spleen cells are incubated with irritation under a proper condition [Y. Tanaka et al, Microbiol. Immunol., 25, 1077 (1981)]. According to this method, the interleukin of mice or rats can be produced in a yield sufficient to conduct the experiment on a test animal level, but in the case of human being, it is extremely difficult to obtain the spleen cells. Therefore, the human peripheral leukocytes have been used in place of the spleen cells, but the yield of interleukin is very poor. For example, interleukin-2 is produced at a rate of 0.01 - 0.05 unit per 1 x $10^6$ cells of the peripheral blood. This value is extremely low since it is possible that interleukin-2 is produced by the spleen cells of mice or rats at a rate of 20 - 30 units per 1 x $10^6$ cells. It has not yet been elucidated why the yield of interleukin is so low.

Summary of the Invention

It has now been found by the present inventor that interleukin-2 and macrophage activating factor are produced by the human peripheral blood cells in a very high concentration on incubation in the presence of the human lymphoblast cells and an interleukin inducer. The present invention is based on this finding. Thus resulting interleukin-2 and macrophage are greatly useful in treatment of infectious diseases caused by virus or bacteria or of various types of malignant tumors. Furthermore, they enhance the preventive or therapeutic effect of other lymphokines including interferon in combined use of them.

## Description of the Preferred Embodiment

The human peripheral blood cell may be a monocyte component mainly consisting of the lymphocyte and macrophage which are separated from the heparin-added blood by Ficoll-Isopaque density gradient centrifugation, or alternatively it may be total cell component (including polymorphonuclear leukocytes) separated by lymphopheresis.

On the other hand, as the lymphoblast cell, it is appropriate to use the lymphoblast cell of B cell type, particularly BALL-1 cell, which has been isolated from the human acute lymphoblastic leukemia and confirmed to be derived from the B lymphocyte and to have surface immunoglobulin [I, Miyoshi et al., Nature, 267, 843 (1977)]. The cell has been adapted to an RPMI-1640 medium containing 10 % bovine fetal serum on which the cell grows; for example, in order to secure the live cell, the cell may be subcultured 2 - 3 times a week on an RPMI-1640 medium containing 5 - 10 % bovine fetal or calf sera. The cell may be preserved in an ultra-low temperature container at -80°C, and revived in a state possible for incubation when demanded.

The human lymphoblast cell in addition to the aforementioned one may be of other B cell type ones, particularly other subline cell derived from the BALL-1 cell, or of the T cell type ones. These human lymphoblast cells have widely been known to the reserchers in this field, and are also obtainable by cell formation by incubation.

The interleukin inducers employed in the present invention mean plant agglutinins, bacterial toxins and enzymes, all of which are known as interleukin inducer. The plant agglutinins include concanavalin A (Con A), phytohemagglutinin (PHA), etc.; the bacterial toxins include staphylococcal enterotoxin (SEA), etc. The preferred enzyme is neuraminidase (NA) or galactose oxidase. Additionally, substances which are generally used in induction of γ-interferon exhibit the effect as interleukin inducers.

The process of the present invention may be carried out by mixing the human peripheral blood cell with the human lymphoblast cell at a proper ratio, adding an interleukin inducer thereto, and incubating the mixture under a proper condition. As for the culture medium it is appropriate to use an RPMI-1640 medium containing usually 2 - 5 % calf or bovine fetal sera. It is also possible to employ Dulbecco's MEM medium and other conventional media for tissue culture. The incubation may preferably be conducted under 5 - 8 % $CO_2$ atmosphere at 35 - 37°C for 12 - 48 hours. The purpose of the incubation may also be attained outside of the above defined scope.

In mixing the human peripheral blood cells with the human lymphoblast cell, the latter (e.g. BALL-1 cell) may be used in an amount of $1 \times 10^5$ - $2.5 \times 10^5$ cell/ml to $1 \times 10^6$ - $2 \times 10^6$ cell/ml of the former. The mixing ratio, however, does not necessarily have an absolute meaning. The preferred concentration of the interleukin inducers has already been determined in each case, and may be applied to the present invention. For example, Con A may be used in a concentration of 2.5 - 5 mcg/ml. When the inducer acts or may possibly act as an inhibitor to culture tissue or in production of interleukin, it may be used for a prefixed relatively short period of time and then removed from the culture medium, and the culture is continued in the absence of the inducer. For example, when NA and GO are employed, they are added to the medium at a rate of about 0.05 unit/ml and about 2 unit/ml, respectively, and the mixture is stirred well and incubated at about 37°C for about 30 minutes; the suspended cells are collected and washed, to which added another medium, and the incubation is continued further in the same condition as mentioned above.

The objective interleukin-2 and macrophage activating factor are released in the culture medium; after termination of the incubation, the cells are removed by centrifugation or other operation, and the supernatant is worked up by means of conventional manners such as precipitation, dialysis, gelfiltration, liquid chromatography, high performance liquid chromatography, or affinity chromato- graphy for purification and concentration, and then lyophilized to yield interleukin-2 and macrophage activating factor as high potency products. In practice it is not necessary to separate those products each other.

The advantage of the present invention is that the productivity of interleukin-2 is markedly increased when the incubation of the human peripheral blood cells stimulated by an interleukin inducer is carried out in the presence of the human lymphoblast cells. For example, the yield of interleukin-2 from 1L of the culture broth containing $1 \times 10^6$ cell/ml of the human peripheral blood cells is only about 30 unit on the stimulation with Con A. On the contrary, the yield of interleukin-2 reaches 20,000 - 30,000 unit or more in the presence of BALL-1 cells. Similarly, the yield of macrophage activating factor is increased by about 10 times.

The present invention will be explained further by the following examples, which are not intended as a limitation thereof. In the examples, the identification and assay of the objective compound interleukin-2 were effected according to the method developed by the present inventor [Kumagai et al., Conference of the Japanese

Society of Immunology, 11th, 277 (1981)]. Other assay
methods are also applicable [T. Shaw et al., J. Immunol.,
120, 1967 (1978)].

Example 1

In 100 ml of an RPMI-1640 medium is suspended the
peripheral mononuclear cells (about $1 \times 10^8$) at a rate of
$1 \times 10^6$ cell/ml which is isolated from 100 ml of the
human peripheral blood containing heparin by means of the
Ficoll-Isopaque density gradient centrifugation. BALL-1
cells which have previously been cultured and grown are
admixed therewith at a rate of $2 \times 10^5$ cell/ml, and then
Con A is added thereto at a concentration of 25 mcg/ml
and mixed well. Then, 2 % bovine fetal serum is added
thereto, and the mixture for culture 20 ml each is placed
in a 100 ml bottle and incubated at 37°C in an incubator
containing 8 % $CO_2$ under a stationary condition for 48
hours, during which time the cells are agitated occasionally
in the bottle. The ratio of the cell suspension to the
upper air in the bottle is about 2 : 8 by volume.

After termination of the incubation, the cells are
removed by centrifugal separation at 1,500 rpm for 5
minutes at room temperature (20°C). The resulting supernatant collected is filtered through a Millipore filter of
0.45 mµ, and the content of interleukin in the filtrate is
determined. As a reference experiment for comparison, the same operation is made in the absence of BALL-1 cells to yield the filtrate of culture broth.

The activity of interleukin-2 was determined as follows. The DNA synthesis by natural killer (NK) clone cell which is derived from the NK cells of mouse spleen and grows dependently in the presence of interleukin-2 is observed on the degree of incorporation of $^3$H-labelled thymidine. The cells of NK clone strain are suspended in an RPMI-1640 medium containing 5 % bovine fetal serum, to which the test sample of each serial dilution is added at 50 % concentration, and incubated at 37°C under 8 % $CO_2$ for 24 hours. Then, 0.5 μ Ci of $^3$H-thymidine is added thereto, and after incubation for 24 hours the radio-activity of $^3$H-thymidine incorporated in the cell is measured by a scintillation counter. One unit is represented by the reciprocal number of dilution concentration of the test sample by which 50 % of the maximum incorporation is incorporated.

The activity of macrophage activating factor (MAF) was determined as follows. THP-1 cells ($5 \times 10^5$) derived from the human monocyte (macrophage) [S. Tsuchiya et al., Int. J. Cancer, 26, 171 (1980)] are incubated on an RPMI-1640 medium containing 10 % calf serum, to which after the lapse of 1 day the test sample of each serial dilution is added at 50 % concentration, and further incubated at 37°C for 5 days.

MAF activates the THP-1 cell and gives it a phagocytotic activity, which may be determined as follows. To the cell which has been cultured for 5 days is added 1 % heat-treated yeast, and the phagocytic rate is determined. One unit is represented by the reciprocal number of dilution concentration of the test sample which shows 50 % of the maximum phagocytic rate.

The result is shown as follows.

Control:

    Interleukin-2                            3 unit/100ml

    Macrophage activating factor      400 unit/100ml

In the present invention:

    Interleukin-2                      2560 unit/100ml

    Macrophage activating factor     3200 unit/100ml

The supernatant of the culture broth showing the interleukin-2 activity is purified by Sephacryl S-200 chromatography by which the active fraction is separated from concomitant proteins.

The supernatant of the culture broth is condensed with Amicon YM-10 to 3 ml, which is poured into Sephacryl S-200 (20 x 2.4 cm) preliminarily equilibrated with 0.15M-phosphate buffer and developed with the same buffer (20 - 30 ml/hour) at 4°C. As shown in Fig 1 (each fraction, 4 ml), interleukin-2 is separated from two kinds of the main

concomitant proteins and eluted in the predetermined fractions of approximate molecular weight 15,000. The content of interluekin-2 per unit volume is concentrated up to about 100 times by this purification procedure.

Example 2

In 40 ml of an RPMI-1640 medium is suspended the peripheral mononuclear cells (about $1 \times 10^8$) at a rate of $1 \times 10^6$ cell/ml which is isolated from 100 ml of the human peripheral blood by means of the Ficoll-Isopaque density gradient centrifugation. BALL-1 cells which have previously been cultured and grown are admixed therewith at a rate of $2 \times 10^5$ cell/ml. Then, NA and GO are added thereto at a rate of 0.05 unit/ml and 2 unit/ml, respectively, and the mixture is warmed at 37°C for 30 minutes, and centrifuged at 1,500 rpm at room temperature (20°C) for 5 minutes. The separated cells are washed and suspended in an RPMI medium containing 5 % calf serum at a rate of $1 \times 10^6$ cells/ml. The suspension is divided into 10 ml each and incubated in an 8 % $CO_2$-incubator at 37°C for 48 hours. After termination of the incubation, the cells are removed by centrifugation at 1,500 rpm for 5 minutes at room temperature. The supernatant collected is filtered through a Milipore filter of 0.45 mµ, and the content of interleukin-2 is determined.

As a reference experiment for comparison, the same operation is made in the absence of BALL-1 cell to yield the filtrate of culture broth. The result is shown as follows.

Control:

    Interleukin-2                   30 unit/100ml

In the present invention:

    Interleukin-2                   10,640 unit/100ml

In the same way as above, the human peripheral blood ($1 \times 10^6$ cells/ml) was incubated with Con A (5 μg/ml) and BALL-1 ($1 \times 10^6$ cells/ml) or with PHA (1 %) and BALL-1 ($1 \times 10^6$ cells/ml) at 37°C under $CO_2$ atmosphere for 48 hours. The results were as follows.

|  | Interleukin-2 (unit/100ml) |
|---|---|
| Con A + BALL 1 | 3,300 |
| PHA + BALL 1 | 2,600 |
| NAGO - BALL 1* | 10,200 |

* NAGO treatment

## Brief Explanation of the drawing

Fig. 1 shows a chromatogram in purification in Example 1. The horizontal axis means the fraction number, the left perpendicular does optical density (OD) measured at 280 nm, and the right perpendicular does the counting (cpm) of radioactivity per minute incorporated in the NK cell.

- 13 -

0094317

What We Claim Is:

(1)   A process for producing interleukin and its analogous immune regulating factors which comprises incubating human peripheral blood cells in the presence of human lymphoblast cells and an interleukin inducer, and recovering interleukin-2 and macrophage activating factor from the culture broth.

(2)   A process claimed in Claim 1, wherein the human peripheral blood cell is mononuclear cell component containing human lymphocyte and macrophage.

(3)   A process claimed in Claim 1, wherein the human lymphoblast cell is B lymphoblast cell.

(4)   A process claimed in Claim 1, wherein the interleukin inducer is a plant agglutinin or bacterial toxin or enzyme.